# EUROPEAN PATENT APPLICATION

(11) **EP 1 785 138 A1**
(43) Date of publication of application: **16.05.2007**
(21) Application number: 05780925.3
(22) Date of filing: 25.08.2005
(51) Int. Cl.: A61K 31/4745, A61K 31/047, A61K 31/122, A61K 47/10, A61K 47/12, A61K 47/22, A61K 47/42, A61P 3/10, A61P 25/14

(54) **MITOCHONDRIA ACTIVATORS**

(30) Priority: 30.08.2004 JP 2004249494; 30.08.2004 JP 2004249495
(71) Applicant: KANEKA CORPORATION, Osaka-shi, Osaka-fu (JP)
(72) Inventor: FUJII, Kenji, Kobe-shi, Hyogo 651-1202 (JP); MATSUMOTO, Shuka, Himeji-shi, Hyogo 672-8012 (JP); HOSOE, Kazunori, 676-0025 (JP)
(74) Representative: Hart Davis, Jason
(86) International application number: PCT/JP2005/015401
(87) International publication number: WO 2006/025247

(57) **Abstract**

The present invention provides a mitochondrial activator containing pyrroloquinoline quinone, preferably pyrroloquinoline quinone and coenzyme Q (particularly reduced coenzyme Q) as active ingredients. In addition, the present invention provides a mitochondrial activator containing citric acid, preferably citric acid and coenzyme Q (particularly reduced coenzyme Q), as active ingredients.

Since pyrroloquinoline quinone or citric acid exhibits a synergistic mitochondria activating effect with coenzyme Q (particularly reduced coenzyme Q), a high effect can be obtained even at a low dose. According to the present invention, therefore, a safer and practical mitochondrial activator is provided.

The present invention further provides an agent for the prophylaxis or treatment of a disease caused by mitochondrial dysfunction.

## Description

The present invention relates to a mitochondrial activator containing a pyrroloquinoline quinone derivative as an active ingredient. The present invention also relates to a mitochondrial activator containing reduced coenzyme Q and/or oxidized coenzyme Q, and a citric acid derivative as active ingredients. As used herein, the mitochondrial activator is a composition capable of preventing or improving the symptoms of various diseases relating to the attenuation or failure of mitochondrial function, for example, diabetes, mitochondrial disease and brain disease.

### Background Art

Mitochondria are intracellular organelles, known to be involved in various metabolisms and energy production. Particularly, adenosine triphosphate (ATP) important for the biological energy is a main resultant product of mitochondria. Low mitochondrial activity directly leads to the depletion of ATP, and depletion of ATP develops various diseases. For example, heart diseases, myasthenia, diabetes, liver diseases, mitochondrial diseases (mitochondrial dysfunction), brain diseases (e.g., neurodegenerative diseases such as Parkinson's disease, Huntington's disease, Alzheimer's disease, amyotrophic lateral sclerosis and the like, and the like) can be caused, of which diabetes is particularly important.

Diabetes is a most common and significant lifestyle-related disease, and not less than one million patients are suffering from this disease worldwide. Moreover, would-be diabetic patients showing relatively high blood glucose levels far outnumber the diabetic patients. As shown above, diabetes is a serious disease all over the world. Diabetes is known to include type I diabetes caused by inadequate insulin secretion (insulin dependent diabetes), and type II diabetes caused by low sensitivity to insulin (non-insulin dependent diabetes). Many of the diabetic patients have type II diabetes, and this type of diabetes is often developed by an inappropriate lifestyle such as excessive eating and the like. It is considered that type II diabetes is developed by a combination of inadequate insulin secretion from the pancreatic islets of Langerhans and low insulin sensitivity of various tissues. Insulin secretion from the pancreatic islets of Langerhans is triggered by the biosynthesis of ATP in response to an increased blood glucose level. Accordingly, mitochondrial dysfunction is known to suppress insulin secretion and become one cause of diabetes. In recent years, it has been clarified that mitochondrial gene abnormality develops diabetes.

Coenzyme Q is an essential component widely distributed in living organisms from bacteria to mammals, and is known to be an electron transport chain constituting component of intracellular mitochondria in living organisms. Coenzyme Q functions as a transport component in the electron transport chain by repeating oxidation and reduction in mitochondria, and reduced coenzyme Q is known to show an antioxidative action. In humans, coenzyme Q₁₀ is a main component, which is a coenzyme Q having a side chain consisting of 10 repeat units.

An important characteristic of coenzyme Q₁₀ is its high safety. In the chronic toxicity test in rats, the absence of toxic effect has been reported even by consecutive administration of 1200 mg/Kg/day for 52 weeks (non-patent reference 1).

As for coenzyme Q₁₀, an oxidized coenzyme Q₁₀ is widely used as a drug for congestive heart failure in Japan and as a health food in Europe and US. While a report has been made that suggests an effect regarding the relationship between the oxidized coenzyme Q₁₀ and diabetes (non-patent reference 2), the effectiveness on diabetic patients by the use of health food has not been actually known. While reduced coenzyme Q₁₀ is an active form of oxidized coenzyme Q₁₀, since it is easily oxidized by oxygen in the air and converted to an oxidized coenzyme Q₁₀, the reduced coenzyme Q₁₀ has not been conventionally used as a product. While we have previously shown that a reduced coenzyme Q₁₀ is highly effective for diabetes as compared to an oxidized coenzyme Q₁₀ (patent reference 1), it has not been clarified at all that a stronger effect can be obtained by a combination with other compound.

Pyrroloquinoline quinone is a substance indicated in 2003 to have a possibility to be a new vitamin, and shows a strong antioxidative activity. As regards use thereof, the usefulness has been reported in promotion of nerve growth factor production (patent reference 2), treatment of diabetic complications (patent reference 3), suppression of melanin production (patent reference 4) and the like. However, strong toxicity to kidney has also been reported (patent reference 5) and it has a problem of what is called a narrow safety margin. As regards the relationship between pyrroloquinoline quinone and mitochondria, while an incident of mitochondrial dysfunction has been reported when a mouse is bred on a pyrroloquinoline quinone-deficient diet (non-patent reference 2), a mitochondrial function-activating activity by pyrroloquinoline quinone has not been known. In addition, while coenzyme Q and pyrroloquinoline quinone are similar to each other in that they both show an antioxidative activity and have a quinone skeleton, there is no finding as to a synergistic action of them.
patent reference 1: WO03/077895
patent reference 2: JP-A-06-211660
patent reference 3: JP-A-06-256191
patent reference 4: JP-A-08-020512
patent reference 5: JP-A-05-78247
non-patent reference 1: K.D. Williams, et al. J. Agric. Food Chem. 47 3756-3763, 1999

cited reference 2: Shimomura, Y. et al. Rinsho to Kenkyu, 58 1349-1352, 1981
non-patent reference 2: R. Rucker, et al. In Biochemical and Molecular Biology of Vitamin B-6 and PQQ-dependent Proteins 61-66, 2000

### Disclosure of the Invention

### Problems to be Solved by the Invention

The present invention aims at providing a composition capable of affording a high effect regarding a mitochondria-activating activity, and expected to show a treatment or prophylactic effect for various diseases (particularly diabetes).

### Means of Solving the Problems

We have intensively studied compositions showing a mitochondria-activating activity and found that a pyrroloquinoline derivative has a mitochondria-activating activity. Furthermore, we have found that a synergistic mitochondria-activating activity can be expressed by combining a reduced coenzyme Q and a pyrroloquinoline quinone derivative. Moreover, we have found that a synergistic mitochondria-activating activity can be expressed by combining an oxidized and/or reduced coenzyme Q and a citric acid derivative.

Accordingly, the present invention provides the following:
[1] A mitochondrial activator comprising pyrroloquinoline quinone or a derivative thereof.
[2] The mitochondrial activator of the aforementioned [1], which comprises
   (a) pyrroloquinoline quinone or a derivative thereof and
   (b) a reduced coenzyme Q represented by the following formula (1);

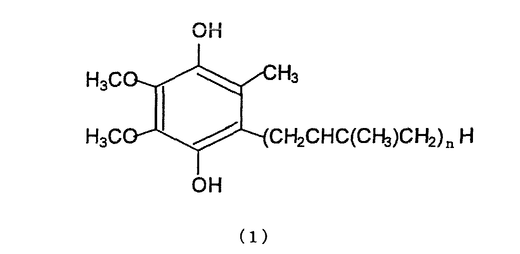

wherein n is an integer of 1 to 12, or (c) an oxidized coenzyme Q represented by the following formula (2);

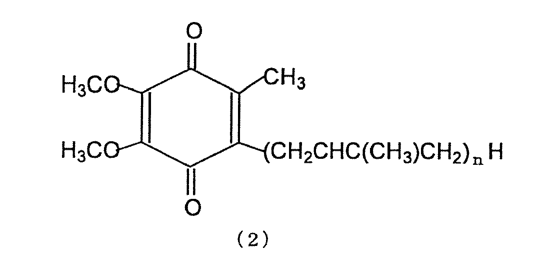

wherein n is an integer of 1 to 12.
[3] A mitochondrial activator comprising at least
   (a) pyrroloquinoline quinone or a derivative thereof, and
   (b) a reduced coenzyme Q represented by the aforementioned formula (1).
[4] The mitochondrial activator of [2] or [3], wherein the coenzyme Q is a coenzyme Q₁₀.
[5] A composition comprising the mitochondrial activator of any one of [1] to [4] and an antioxidant.
[6] The composition of [5], wherein the antioxidant is at least one kind of compound selected from the group consisting of vitamin C, a vitamin C derivative, probucol, lycopene, vitamin A, carotenoids, vitamin B, a vitamin B derivative, flavonoids, polyphenols, glutathione, selenium, sodium thiosulfate, vitamin E, a vitamin E derivative, pycnogenol, flavangenol, citric acid, a citric acid derivative, superoxide dismutase (SOD), glutathioneperoxidase, glutathione-S-transferase, glutathione reductase, catalase and ascorbate peroxidase.
[7] A pharmaceutical or food composition comprising the mitochondrial activator of any one of [1] to [4] and a carrier acceptable as a pharmaceutical agent or food.
[8] A composition for the prophylaxis or treatment of a disease caused by mitochondrial dysfunction, which comprises an effective amount of the mitochondrial activator of any one of [1] to [4].
[9] The composition of [8], wherein the disease is selected from the group consisting of diabetes, a mitochondrial disease and a brain disease.
[10] A method of preventing or treating a disease caused by mitochondrial dysfunction, which comprises administering an effective amount of the mitochondrial activator of any one of [1] to [4] to a mammal.
[11] The method of [10], wherein the disease is selected from the group consisting of diabetes, a mitochondrial disease and a brain disease.
[12] A production method of the mitochondrial activator of any one of the aforementioned [1] to [4], which comprises mixing
   (a) pyrroloquinoline quinone or a derivative thereof, and
   (b) a reduced coenzyme Q represented by the aforementioned formula (1) and/or
   (c) an oxidized coenzyme Q represented by the aforementioned formula (2).
[13] A production method of the composition for the prophylaxis or treatment of the disease caused by mitochondrial dysfunction of the aforementioned [8], which comprises mixing
   (a) pyrroloquinoline quinone or a derivative thereof, and
   (b) a reduced coenzyme Q represented by the aforementioned formula (1) and/or
   (c) an oxidized coenzyme Q represented by the aforementioned formula (2).
[14] A mitochondrial activator comprising
   (b) a reduced coenzyme Q represented by the aforementioned formula (1) or
   (c) an oxidized coenzyme Q represented by the aforementioned formula (2), and
   (d) citric acid or a derivative thereof.
[15] The mitochondrial activator of the aforementioned [14], which comprises at least
   (b) a reduced coenzyme Q represented by the aforementioned formula (1) and
   (d) citric acid or a derivative thereof.
[16] The mitochondrial activator of [14] or [15], wherein the coenzyme Q is a coenzyme Q₁₀.
[17] A composition for a pharmaceutical agent or food, which comprises the mitochondrial activator of any one of [14] to [16], and a carrier acceptable as a pharmaceutical agent or food.
[18] The composition of [17], further comprising an antioxidant substance.
[19] The composition of [18], wherein the antioxidant is one or more kinds of compounds selected from the group consisting of vitamin C, a vitamin C derivative, probucol, lycopene, vitamin A, carotenoids, vitamin B, a vitamin B derivative, flavonoids, polyphenols, glutathione, selenium, sodium thiosulfate, vitamin E, a vitamin E derivative, pycnogenol, flavangenol, pyrroloquinoline quinone, a pyrroloquinoline quinone derivative, superoxide dismutase (SOD), glutathione peroxidase, glutathione-S-transferase, glutathione reductase, catalase, and ascorbate peroxidase.
[20] A composition for the prophylaxis or treatment of a disease caused by mitochondrial dysfunction in a mammal, which comprises an effective amount of the mitochondrial activator of any one of [14] to [16].
[21] The composition of [20], wherein the disease is selected from the group consisting of diabetes, a mitochondrial disease and a brain disease.
[22] A method of preventing or treating a disease caused by mitochondrial dysfunction in a mammal, which comprises administering an effective amount of the mitochondrial activator of any one of [14] to [16] to the mammal.
[23] The method of [22], wherein the disease is selected from the group consisting of diabetes, a mitochondrial disease and a brain disease.
[24] A production method of the mitochondrial activator of any of the aforementioned [14] to [16], which comprises mixing
   (b) a reduced coenzyme Q represented by the aforementioned formula (1) and/or
   (c) an oxidized coenzyme Q represented by the aforementioned formula (2), and
   (d) citric acid or a derivative thereof.
[25] A production method of the composition for the prophylaxis or treatment of the disease caused by mitochondrial dysfunction of the aforementioned [20], which comprises mixing
   (b) a reduced coenzyme Q represented by the aforementioned formula (1) and/or
   (c) an oxidized coenzyme Q represented by the aforementioned formula (2), and
   (d) citric acid or a derivative thereof.
[26] A mitochondrial activator comprising citric acid or a derivative thereof.
[27] A mitochondrial activator comprising
   (a) pyrroloquinoline quinone or a derivative thereof and,
   (b) a reduced coenzyme Q represented by the aforementioned formula (1) and/or
   (c) an oxidized coenzyme Q represented by the aforementioned formula (2), and
   (d) citric acid or a derivative thereof.

### Effect of the Invention

Since lower activity of mitochondria, which is an important organelle to produce biological energy causes various diseases, activation of mitochondrial activity is highly useful for the health. In the present invention, it has been clarified that the pyrroloquinoline quinone derivative itself has a mitochondria-activating activity, and further, that a combination of a reduced coenzyme Q and a pyrroloquinoline quinone derivative affords a synergistic mitochondria-activating activity. Moreover, it has also been clarified that a combination of a reduced coenzyme Q and/or an oxidized coenzyme Q and a citric acid derivative affords a synergistic mitochondria-activating activity. Pyrroloquinoline quinone derivatives give rise to safety problems, and a sufficient amount of citric acid is difficult to take due to the strong sourness it possesses. Since use of these compounds at a low dose affords a synergistic effect, a safer and highly useful composition can be provided.

### Best Mode for Embodying the Invention

The activation of mitochondria in the present invention may mean mitochondrial activation. Specifically, when a certain compound is administered to a test subject and the mitochondrial activity of the test subject becomes higher than that of the control, the compound has a mitochondria-activating activity. The mitochondrial activity can be evaluated, for example, by measuring ATP producing ability.

A first aspect of the present invention is a mitochondrial activator containing pyrroloquinoline quinone or a derivative thereof as an active ingredient.

Pyrroloquinoline quinine is 2,7,9-tricarboxy-1H-pyrrolo[2,3-f]quinoline-4,5-dione. In the present specification, a derivative of a certain compound includes the compound itself, and is produced by a structural change in a small part of the compound. A compound wherein a hydrogen atom or a particular atomic group is substituted by other atom or atomic group is understood to be a derivative of the compound.

As the pyrroloquinoline quinone derivative, for example, pyrroloquinoline quinol, imidazole pyrroloquinoline quinone, an imidazole pyrroloquinoline quinone derivative, a pyrroloquinoline quinone lactone acid salt and the like can be used. Needless to say, it may be pyrroloquinoline quinine *per se.* Of these, pyrroloquinoline quinone, pyrroloquinoline quinone lactone acid salt, pyrroloquinoline quinol and the like are preferable, and pyrroloquinoline quinine is more preferable. As these pyrroloquinoline quinone derivatives, commercially available products may be used as they are, or yeast, dried fungi and food highly containing a pyrroloquinoline quinone derivative may be used. In the present specification, pyrroloquinoline quinone and a derivative thereof may be referred to as pyrroloquinoline quinones.

The mitochondrial activator in the present invention may contain, as an active ingredient besides the above-mentioned pyrroloquinoline quinone or a derivative thereof, a reduced coenzyme Q represented by the following formula (1);

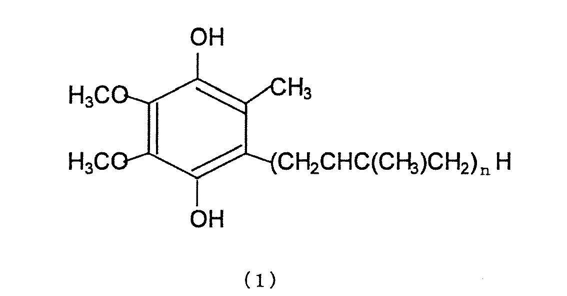

wherein n is an integer of 1 to 12, and/or an oxidized coenzyme Q represented by the following formula (2);

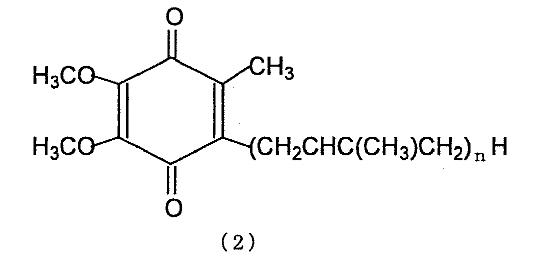

wherein n is an integer of 1 to 12.

It is known that generally about 40-90% of coenzyme Q in living organisms is in a reduced form.

The method for obtaining coenzyme Q is not particularly limited and, for example, it can be obtained by a conventionally known method such as fermentation, chemical synthesis, extraction from naturally occurring substance and the like. The thus-obtained coenzyme Q is mostly oxidized, and the thus-obtained coenzyme Q may be directly utilized as an oxidized coenzyme Q or may be further purified. The method for obtaining a reduced coenzyme Q is not particularly limited and, for example, a method comprising obtaining coenzyme Q by the above-mentioned known method and concentrating a reduced coenzyme Q fraction in the effluent by chromatography and the like can be employed. In this case, where necessary, a general reducing agent such as sodium borohydride, sodium dithionite (sodium hydrosulfite) and the like is added to the above-mentioned coenzyme Q, oxidized coenzyme Q contained in the above-mentioned coenzyme Q is reduced by a conventional method to give a reduced coenzyme Q, and then the reduced coenzyme Q may be concentrated by chromatography. In addition, it can also be obtained by a method comprising reacting existing high purity coenzyme Q (oxidized coenzyme Q) with the above-mentioned reducing agent. Alternatively, a fungus body containing a reduced coenzyme Q, and the like can be used.

The proportion of the reduced form in coenzyme Q can be generally determined by a method comprising quantitating oxidized coenzyme Q and reduced coenzyme Q in a sample by an HPLC system using a UV detector, calculating from the amount ratio or a method comprising calculating the ratio of oxidized coenzyme Q and reduced coenzyme Q from the peak areas by a system incorporating an electrochemical detector into HPLC. Since an oxidized-reduced substance can be specifically measured and the sensitivity is high in the system incorporating an electrochemical detector, the system is highly useful for the measurement of a reduced form present in a trace amount in living organisms or a sample. To be specific, an electrochemical detector manufactured by Shiseido Co., Ltd. was incorporated into an HPLC analysis apparatus manufactured by Shimadzu Corporation, and the measurement was performed under the following HPLC conditions. The conditions were column: YMC-Pack (ODS-A303), detection wavelength: 275 nm, mobile phase: methanol (88%), hexane (12%) and flow rate: 1 ml/min.

In the present specification, a simple indication of "coenzyme Q" means any of the above-mentioned oxidized coenzyme Q, reduced coenzyme Q, and a mixture of oxidized coenzyme Q and reduced coenzyme Q.

In the first mitochondrial activator of the present invention, the contents of the coenzyme Q and pyrroloquinoline quinone derivative are not particularly limited, and can be appropriately determined in view of the product concept and the like. While an increased ratio of reduced coenzyme Q in the coenzyme Q may result in a higher cost due to a stabilizing measure and the like, it can afford a higher synergistic effect with a pyrroloquinoline quinone derivative.

When a combination of two or more active substances provides an effect exceeding the effect afforded by each of the substances or addition of respective effects (i.e., additive effect), the active substances are considered to have a synergistic effect. A combination of two or more active substances having a synergistic effect can form a combination agent capable of affording a synergistic effect. The term "synergistic" used in the present specification means that an activity afforded by an interaction between a first active component and a second active component (and when present, a third active component) is higher than the activity expected based on the actions observed when the components are applied separately. Therefore, a significantly higher activity than the addition of the effects of individual active components can be obtained by the concurrent application. Accordingly, the present invention also relates to a synergistic composition. The synergistic composition of the present invention can achieve an effect equivalent to or higher than the effect provided by a single application of each active component, at a dose lower than the effective amount of each active component, namely, a synergistically effective amount.

Alternatively, in the present invention, the pyrroloquinoline quinone derivative is considered to potentiate the mitochondria-activating activity of coenzyme Q (and vice versa), when combined with reduced coenzyme Q and/or oxidized coenzyme Q. Therefore, an enhancer of a mitochondrial activator containing coenzyme Q, which enhancer comprising a pyrroloquinoline quinone derivative, is another aspect of the present invention. Conversely, an enhancer of a mitochondrial activator containing a pyrroloquinoline quinone derivative, which enhancer comprising coenzyme Q, is another aspect of the present invention. A yeast, dried fungi or food highly containing a pyrroloquinoline quinone derivative can also be used.

The weight ratio of the reduced coenzyme Q or oxidized coenzyme Q and pyrroloquinoline quinone derivative of the present invention is not particularly limited. For example, about 100 to 10, about 10 to 1, about 1 to 0.1, or about 0.1 to 0.01 part by weight, of coenzyme Q can be used per 1 part by weight of a pyrroloquinoline quinone derivative. A desirable weight ratio is about 10:1 to 1:10. A weight ratio of about 1:1 is most desirable. In addition, a pyrroloquinoline quinone derivative alone may be used as the active ingredient.

A second mitochondrial activator of the present invention contains a citric acid derivative as the active ingredient. The activator may contain a citric acid derivative alone as the active ingredient, or may be a composition containing a reduced coenzyme Q represented by the aforementioned formula (1) and/or an oxidized coenzyme Q represented by the aforementioned formula (2), and a citric acid derivative. Since a synergistic mitochondria-activating activity can be afforded, it is preferable to contain a citric acid derivative and coenzyme Q as active ingredients.

As regards reduced coenzyme Q and oxidized coenzyme Q used for the second mitochondrial activator of the present invention, the explanation of the reduced coenzyme Q and oxidized coenzyme Q used for the aforementioned first mitochondrial activator also applies.

In the second mitochondrial activator of the present invention, a commercially available product can be used as citric acid and a citric acid derivative.

The derivative in the present specification is a compound formed by a structural change in a small part of a certain compound. A compound wherein a hydrogen atom or a particular atomic group has been substituted by other atom or atomic group is considered to be a derivative of the compound. In the present specification, the compound itself is also encompassed in the derivative thereof. As the citric acid derivative, citric acid, potassium citrate, sodium citrate, calcium citrate, isopropyl citrate, iron citrate and the like are concretely used. A preferable citric acid derivative is sodium citrate.

In the second mitochondrial activator of the present invention, the contents of the coenzyme Q and the citric acid derivative are not particularly limited, and can be appropriately determined in view of the product concept and the like. While an increased ratio of reduced coenzyme Q in the coenzyme Q may result in a higher cost due to a stabilizing measure and the like, it can afford a higher synergistic effect with a citric acid derivative.
As regards the synergistic effect here, the above explanation of the first mitochondrial activator also applies.

Alternatively, in the present invention, the citric acid derivative is considered to potentiate the mitochondria-activating activity of coenzyme Q, when combined with reduced coenzyme Q and/or oxidized coenzyme Q. Therefore, an enhancer of a mitochondrial activator containing coenzyme Q, which enhancer comprising a citric acid derivative, is another aspect of the present invention. Conversely, an enhancer of a mitochondrial activator containing a citric acid derivative, which enhancer comprising coenzyme Q, is another aspect of the present invention. A yeast, dried fungi or food highly containing a citric acid derivative can also be used. The weight ratio of the coenzyme Q and citric acid derivative of the present invention may be, for example, 1:100 to 10:1. The weight ratio is desirably, for example, about 1:1, more desirably about 1:10, which is not particularly limited.

Moreover, the present invention provides a composition for mitochondrial activation, which comprises
(a) pyrroloquinoline quinone or a derivative thereof, and
(b) a reduced coenzyme Q represented by the following formula (1);

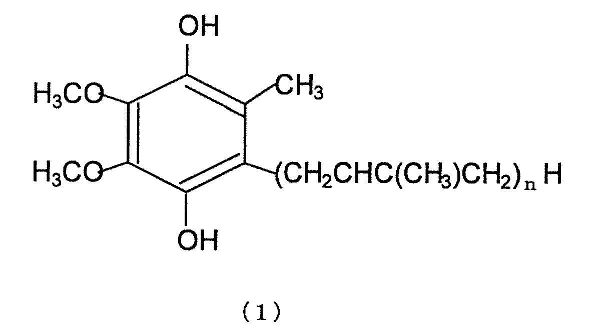

wherein n is an integer of 1 to 12, and/or (c) an oxidized coenzyme Q represented by the following formula (2);

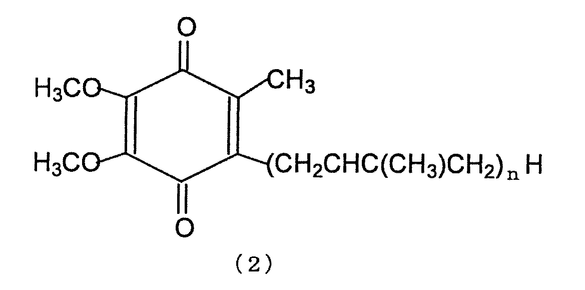

wherein n is an integer of 1 to 12, and
(d) citric acid or a derivative thereof. Such various components are as explained above.

The following is an explanation common to the first mitochondrial activator and the second mitochondrial activator of the present invention. When simply referred to as an "active ingredient", it means a pyrroloquinoline quinone derivative, preferably a pyrroloquinoline quinone derivative and a coenzyme Q in the first mitochondrial activator, and a citric acid derivative, preferably a citric acid derivative and a coenzyme Q, in the second mitochondrial activator. In the mitochondrial activator of the present invention, as coenzyme Q, oxidized coenzyme Q or reduced coenzyme Q alone may be used as the active ingredient, or a mixture of reduced coenzyme Q and oxidized coenzyme Q may be used as the active ingredient. Since a higher synergistic effect can be obtained with pyrroloquinoline quinone or a citric acid derivative, it is preferable that coenzyme Q contain at least a reduced coenzyme Q. While the ratio of the reduced coenzyme Q and the oxidized coenzyme Q is not particularly limited, the ratio of the reduced coenzyme Q is preferably not less than about 60 wt% and not more than about 100 wt%, more preferably not less than about 80 wt% and not more than about 99 wt%, relative to the whole coenzyme Q.

As the reduced coenzyme Q usable in the present invention, those represented by the aforementioned formula (1) wherein the side chain has 1 to 12 repetitive units (n in the formula) can be used. Of these, one having 10 repetitive units in the side chain, or a reduced coenzyme Q₁₀, can be used particularly preferably. An oxidized coenzyme Q₁₀ can also be preferably used as the oxidized coenzyme Q represented by the aforementioned formula (2).

The mitochondrial activator of the present invention can be formulated into a desired form. The mitochondrial activator of the present invention may contain the active ingredient of the present invention (may be two or more kinds) in a proportion of about 0.1 to 100 wt%, preferably about 1 to 99 wt%, more preferably about 10 to 90 wt%. The dosage form of the mitochondrial activator of the present invention is not particularly limited, and may be an oral preparation or directly applied to the skin. For example, the oral preparation may be a powder, or granules containing a binder, or a capsule obtained by filling a capsule with a powder or granules. In addition, natural oil, oily higher fatty acid, higher fatty acid monoglyceride, surfactant or a mixture thereof and the like may be added and the mixture is filled in an oil state to give a soft capsule. In this case, one mainly containing gelatin, one mainly containing other water-soluble polymer substance and the like can also be used. Such capsule also includes microcapsules. Alternatively, the preparation may be formed into a liquid and used as a drinkable agent.

The mitochondrial activator of the present invention may further contain, in addition to the above-mentioned active ingredient, other preparation carriers that are pharmaceutically acceptable or acceptable for food engineering, as appropriate according to a conventional method. Such carriers are not particularly limited and, for example, excipient, disintegrant, lubricant, binder, antioxidant, coloring agent, anticoagulant, absorption promoter, dissolution aids, stabilizer and the like can be mentioned.

The above-mentioned excipient is not particularly limited and, for example, sucrose, lactose, glucose, cornstarch, mannitol, crystalline cellulose, calcium phosphate, calcium sulfate, and the like can be mentioned. The above-mentioned disintegrant is not particularly limited and, for example, starch, agar, calcium citrate, calcium carbonate, sodium hydrogencarbonate, dextrin, crystalline cellulose, carboxymethylcellulose, tragacanth and the like can be mentioned.

The above-mentioned lubricant is not particularly limited and, for example, talc, magnesium stearate, polyethylene glycol, silica, hardened vegetable oil and the like can be mentioned. The above-mentioned binder is not particularly limited and, for example, ethylcellulose, methylcellulose, hydroxypropylmethylcellulose, tragacanth, shellac, gelatin, gum arabic, polyvinylpyrrolidone, polyvinyl alcohol, polyacrylic acid, polymethacrylic acid, sorbitol and the like can be mentioned.

The above-mentioned antioxidant is not particularly limited and, for example, ascorbic acid, tocopherol, vitamin A, β-carotene, sodium bisulfite, sodium thiosulfate, sodium pyrosulfite, citric acid and the like can be mentioned.

The above-mentioned coloring agent is not particularly limited and, for example, those permitted for addition to pharmaceutical products and the like can be used.

The above-mentioned anticoagulant is not particularly limited and, for example, stearic acid, talc, light anhydrous silicic acid, hydrated silicon dioxide and the like can be mentioned.

The above-mentioned absorption promoter is not particularly limited and, for example, surfactants such as higher alcohols, higher fatty acids, glycerin fatty acid ester, sucrose fatty acid ester, sorbitan fatty acid ester, polyoxyethylene sorbitan fatty acid ester, polyglycerin fatty acid ester and the like, and the like can be mentioned.

The above-mentioned dissolution aids is not particularly limited and, for example, organic acids such as fumaric acid, succinic acid, malic acid and the like, and the like can be mentioned. The above-mentioned stabilizer is not particularly limited and, for example, benzoic acid, sodium benzoate, ethyl parahydroxybenzoate and the like can be mentioned. For direct application to the skin, the dosage form is not particularly limited and, for example, those prepared in the form of cream, paste, jelly, gel, emulsion or liquid (ointment, liniment, lotion, cream, spray and the like) by dissolving or dispersing, in a mixture, the above-mentioned drug in a suitable base, one wherein a base containing the above-mentioned drug dissolved or dispersed, in a mixture, therein is flattened on a support (cataplasm and the like), one wherein an adhesive containing the above-mentioned drug dissolved or dispersed, in a mixture, therein is flattened on a support (plaster, tape and the like) and the like can be mentioned. As the base and adhesive, bases generally used for pharmaceutical products, cosmetic and the like can be used as necessary as long as the effect of the present invention is not impaired.

The mitochondrial activator of the present invention can concurrently contain an antioxidant, for example, an antioxidant substance and an antioxidant enzyme. While the antioxidant substance is not particularly limited, for example, vitamin E, vitamin E derivative, vitamin C, vitamin C derivative, probucol, lycopene, vitamin A, carotenoids, vitamin B, vitamin B derivative, flavonoids, polyphenols, glutathione, pycnogenol, flavangenol, citric acid, citric acid derivative, pyrroloquinoline quinone, pyrroloquinoline quinone derivative, selenium and the like are suitable. The above-mentioned substances may be used alone or in a mixture of two or more kinds. While the antioxidant enzyme is not particularly limited, for example, superoxide dismutase (SOD), glutathioneperoxidase, glutathione-S-transferase, glutathionereductase, catalase, ascorbate peroxidase and the like are suitable. The above-mentioned substances may be used alone or in a mixture of two or more kinds.

The mitochondrial activator of the present invention can concurrently contain other nutritious and tonic components. While the nutritious and tonic component is not particularly limited, for example, creatine, taurine, vitamin B₁, vitamin B derivative, amino acid and the like are suitable. The above-mentioned substances may be used alone or in a mixture of two or more kinds. By mixing the active ingredient of the present invention with these components, an additive or synergistic effect can be further expected.

The mitochondrial activator of the present invention can concurrently contain a nutritious supplement component. While the nutritious supplement component is not particularly limited, amino acid, metal ion, saccharides, proteins, fatty acids, vitamin and the like can be mentioned.

While the form of the mitochondrial activator of the present invention when it is prepared into a general food is not particularly limited, edible oil and fat composition, cooking oils, spray oils, butters, margarines, shortenings, whip creams, concentrated milks, whiteners, dressings, pickle liquids, breads, cakes, pies, cookies, Japanese confectioneries, snacks, fried confectioneries, chocolate and chocolate confectioneries, rice confectioneries, rouxs, sauces, bastes, toppings, ice confectioneries, noodles, bakery mixes, fried foods, processed meat products, fish paste products, frozen foods such as frozen entrees, livestock frozen food, agricultural frozen food and the like, rice diets, jams, cheese, cheese food, cheese-like food, gums, candies, fermented milks, cans, beverages and the like can be mentioned.

The mitochondrial activator of the present invention can improve the mitochondrial activity in the skin by directly applying to the skin. In this case, by the activation of skin cells, an improving effect on the skin turgor, moisture, wrinkle, dullness, fleck and the like can be afforded. Moreover, a component aiming at the above-mentioned skin care effect can be contained concurrently.

When the mitochondrial activator of the present invention is to be produced, the contents of various active ingredients, product form, preservation method and preservation manner of the product can be appropriately determined according to the design and use and the like of the commercial products of the mitochondrial activator.

The mitochondrial activator of the present invention can afford an improvement effect on the disease, poor physical condition or skin care, which involves mitochondria. The disease caused by mitochondrial dysfunction includes various diseases relating to the attenuation or failure of mitochondrial function. The diseases include, for example, diabetes, skin disease (e.g., rough skin), fatigue (e.g., chronic fatigue syndrome), heart disease, mitochondrial disease and brain disease. The mitochondrial activator of the present invention can be used as a composition for the treatment, prophylaxis, medical treatment or improvement of the above-mentioned disease or conditions.

The subject mammals also include those other than human, but human is preferable. The "effective amount" means an amount of a compound that confers a prophylactic or therapeutic effect to the subject of medical treatment. The therapeutic effect could be objective (i.e., measurable by some test or marker) or subjective (i.e., pointed out or felt by the subject).The dose level of the aforementioned compound and administration frequency of the combination vary depending on the ability of each compound to be used, metabolic stability and duration of action of the compound, age, body weight, general health and sex of patients, diet, manner and timing of administration, clearance rate, drug combination, severity of the condition to be treated, and treatment or prevention that patients undergo. The determination of the preferable effective amount is within the general technical scope of those of ordinary skill in the art, and those of ordinary skill in the art can empirically or experimentally determine such amount.

Noting a reduced and/or oxidized coenzyme Q as an active ingredient, the daily dose is, for example, about 10 mg to about 500 mg, preferably about 30 mg to about 300 mg, particularly about 50 to about 100 mg, per human for one day, which is administered at once or multiple times. The daily dose of the pyrroloquinoline quinone derivative or citric acid derivative, which are the other active ingredients, is similar to that of the above-mentioned reduced and/or oxidized coenzyme Q. In general, oral administration is employed but, for example, administration by way of transdermal, transmucosal or enteral administration, or injection such as direct infusion into the blood vessel and the like can also be employed.

The present invention provides a combination agent wherein the first action component and the second action component, each in a synergistically effective amount, are each formed into an individual unit, such as capsule, tablet or pill, and then combined with each other. The present invention also provides a commercial product comprising the combination agent together with, where necessary, an instruction sheet indicating that they should be simultaneously or sequentially applied, or a packaging with an indication to that effect.

The production method of the first mitochondrial activator of the present invention includes mixing a reduced coenzyme Q and/or an oxidized coenzyme Q with a pyrroloquinoline quinone derivative. Where desired, an antioxidant and the aforementioned preparation carrier and the like may be admixed. Pharmaceutically or food engineeringly applicable production methods can be combined. In a similar manner, a composition for a pharmaceutical agent or food can be produced.

The production method of the second mitochondrial activator of the present invention includes mixing a reduced coenzyme Q and/or an oxidized coenzyme Q with a citric acid derivative. Where desired, an antioxidant and the aforementioned preparation carrier and the like may be admixed. In a similar manner, a composition for a pharmaceutical agent or food can be produced. Pharmaceutically or food engineeringly applicable production methods can be combined.

### Examples

The present invention is explained in more detail in the following by referring to Examples and Formulation Examples, which are not to be construed as limitative.

### (Production Example 1) Production of reduced coenzyme Q₁₀

To 1000 g of ethanol were added 100 g of oxidized coenzyme Q₁₀ (purity 99.4%) and 60 g of L-ascorbic acid and the mixture was stirred at 78°C to carry out a reduction reaction. After 30 hr, the mixture was cooled to 50°C, and ethanol (330 g) and water (70 g) were added while maintaining the same temperature. The ethanol solution (containing 100 g of reduced coenzyme Q₁₀) was cooled to 2°C at a cooling rate of 10°C/hr with stirring to give a white slurry. The obtained slurry was filtrated under reduced pressure, and the wet crystals were successively washed with cold ethanol, cold water and cold ethanol (temperature of solvent used for washing was 2°C), and further vacuum dried (20-40°C, 1-30 mmHg) to give white dry crystals (97 g). All operations except vacuum drying were performed under a nitrogen atmosphere.

### (Production Example 2) Production of reduced coenzyme Q₁₀

An oxidized coenzyme Q₁₀ (100 g) was dissolved in 1000 g of a heptane solution at 25°C. Under stirring, as a reducing agent, an aqueous solution of sodium hyposulfite (purity 75% or above, 100 g) in water (1000 ml) was gradually added to carry out a reduction reaction at 25°C, pH 4-6. After 2 hr, the aqueous phase was removed from the reaction mixture and the heptane phase was washed 6 times with deaerated saturated saline (1000 g). All the above operations were performed under a nitrogen atmosphere. The solvent of the heptane phase was substituted under reduced pressure and a solution (50°C) of reduced coenzyme Q₁₀ in 7% (w/w) ethanol was prepared (containing 100 g of reduced coenzyme Q₁₀). Water (50 g) was added to the ethanol solution and the mixture was cooled to 2°C at a cooling rate of 10°C/hr with stirring to precipitate crystals. All the operations were performed under a nitrogen atmosphere. The obtained slurry was filtrated under reduced pressure, and the wet crystals were successively washed with cold ethanol, cold water and cold ethanol (temperature of solvent used for washing was 2°C), and further vacuum dried (20-40°C, 1-30 mmHg) to give white dry crystals (97 g).

(Example 1) Effect of the first mitochondrial activator of the present invention on insulin secretion activity A problem was noted in that changes in the production amount of ATP, which is an index of the mitochondrial activity, were difficult to know during evaluation of the mitochondria-activating activity of a test substance, since ATP's intracellular production and consumption proceed almost simultaneously. Therefore, a tissue of the pancreatic islets of Langerhans, which reacts with ATP to secrete insulin, was used to evaluate the mitochondria-activating activity of a test substance, with the insulin secretion as an index.

As the tissue of the pancreatic islets of Langerhans, a culture kit (manufactured by Hokudo Co., Ltd) prepared from a rat was used. The tissue of the islets of Langerhans was precultured in a CO₂ incubator for 24 hr, the medium was changed to one containing test substances (oxidized coenzyme Q₁₀, reduced coenzyme Q₁₀ and pyrroloquinoline quinone) alone or in a mixture and, after culture for 1 hr, insulin secreted in the medium was quantitated by ELISA to evaluate the effect of the test substance on the ATP production. The results thereof are shown in Table 1.

**Table 1 Secretion amount of insulin from tissue of the islets of Langerhans**

| sample | insulin concentration (ng/ml) |
|---|---|
| control | 43±8 (100) |
| oxidized coenzyme Q10 (0.1 µg/ml) | 329±85 (765) |
| reduced coenzyme Q10 (0.1 µg/ml) | 360±78 (837) |
| pyrroloquinoline quinone (0.1 µg/ml) | 220±56 (511) |
| oxidized coenzyme Q10 (0.1 µg/ml) + pyrroloquinoline quinone (0.1 µg/ml) | 1513±362 (3518) |
| reduced coenzyme Q10 (0.1 µg/ml) + pyrroloquinoline quinone (0.1 µg/ml) | 1870±297 (4349) |

The oxidized coenzyme Q₁₀, reduced coenzyme Q₁₀ and pyrroloquinoline quinone in an addition amount of 0.1 µg/ml increased the concentration of insulin in the medium to 7.7-fold, 8.3-fold and 5.1-fold respectively, by which it has been clarified that they activated the ATP production in the tissue of the pancreatic islets of Langerhans. Moreover, the concurrent use of pyrroloquinoline quinone and oxidized or reduced coenzyme Q₁₀ increased the insulin amount to 35-fold and 43-fold, respectively, and it has been clarified that these compounds synergistically act on the biosynthesis of ATP.

### (Example 2) Effect of the second mitochondrial activator of the present invention on insulin secretion activity

In the same manner as in Example 1, a tissue of the pancreatic islets of Langerhans was used to evaluate the mitochondria-activating activity of a test substance, with the insulin secretion as an index. As the tissue of the pancreatic islets of Langerhans, a culture kit (manufactured by Hokudo Co., Ltd) prepared from a rat was used. The tissue of the islets of Langerhans was precultured in a CO₂ incubator for 24 hr, the medium was changed to one containing test substances (oxidized coenzyme Q₁₀, reduced coenzyme Q₁₀ and citric acid) alone or in a mixture and, after culture for 1 hr, insulin secreted in the medium was quantitated by ELISA to evaluate the effect of the test substance on the ATP production. The results thereof are shown in Table 2.

**Table 2 Secretion amount of insulin from tissue of the islets of Langerhans**

| sample | insulin concentration (ng/ml) |
|---|---|
| control | 43±8 (100) |
| oxidized coenzyme Q10 (0.1 µg/ml) | 329±85 (765) |
| reduced coenzyme Q10 (0.1 µg/ml) | 360±78 (837) |
| citric acid (0.1 µg/ml) | 2030±391 (4720) |
| oxidized coenzyme Q10 (0.1 µg/ml) + citric acid (0.1 µg/ml) | 2960±620 (6884) |
| reduced coenzyme Q10 (0.1 µg/ml) + citric acid (0.1 µg/ml) | 3300±480 (7674) |

The oxidized coenzyme Q₁₀, reduced coenzyme Q₁₀ and citric acid in an addition amount of 0.1 µg/ml increased the concentration of insulin in the medium to 7.7-fold, 8.3-fold and 47-fold respectively, by which it has been clarified that they activated the ATP production in the tissue of the pancreatic islets of Langerhans. Moreover, the concurrent use of citric acid and oxidized or reduced coenzyme Q₁₀ increased the insulin amount to 69-fold and 77-fold, respectively, and it has been clarified that these compounds synergistically act on the biosynthesis of ATP.

### (Formulation Example 1)

Olive oil was heated to 60°C, and coenzyme Q₁₀ (reduced coenzyme Q₁₀:oxidized coenzyme Q₁₀=85:15) similarly melted at 60°C and pyrroloquinoline quinone were added. Vitamin E was added thereto by small portions to homogenize the mixture, and the mixture was prepared into a soft capsule by a conventional method. A soft capsule preparation containing 20 mg of coenzyme Q₁₀ and 20 mg of pyrroloquinoline quinone in one capsule was obtained.

| | | |
|---|---|---|
| (1) | coenzyme Q₁₀ | 20 parts by weight |
| (2) | pyrroloquinoline quinone | 20 parts by weight |
| (3) | vitamin E | 15 parts by weight |
| (4) | olive oil | 350 parts by weight |

### (Formulation Example 2)

Coenzyme Q₁₀ (reduced coenzyme Q₁₀:oxidized coenzyme Q₁₀=85:15) and pyrroloquinoline quinone were added to acetone, and the mixture was adsorbed to crystalline cellulose (fine powder) and dried. This was mixed with cornstarch, and the mixture was processed to give a powder by a conventional method.

| | | |
|---|---|---|
| (1) | coenzyme Q₁₀ | 10 parts by weight |
| (2) | pyrroloquinoline quinone | 10 parts by weight |
| (3) | crystalline cellulose | 40 parts by weight |
| (4) | cornstarch | 55 parts by weight |

### (Formulation Example 3)

Coenzyme Q₁₀ (reduced coenzyme Q₁₀:oxidized coenzyme Q₁₀=85:15) and pyrroloquinoline quinone were added to acetone, and the mixture was adsorbed to crystalline cellulose (fine powder) and dried. This was mixed with cornstarch, lactose, carboxymethylcellulose and magnesium stearate, an aqueous solution of polyvinylpyrrolidone was added as a binder and the mixture was granulated by a conventional method. Talc was added thereto as a lubricant, mixed, and the mixture was compressed to give a tablet containing 20 mg of coenzyme Q₁₀ and 20 mg of pyrroloquinoline quinone per tablet.

| | | |
|---|---|---|
| (1) | coenzyme Q₁₀ | 20 parts by weight |
| (2) | pyrroloquinoline quinone | 20 parts by weight |
| (3) | cornstarch | 25 parts by weight |
| (4) | lactose | 15 parts by weight |
| (5) | carboxymethylcellulose calcium | 10 parts by weight |
| (6) | crystalline cellulose | 40 parts by weight |
| (7) | polyvinylpyrrolidone | 5 parts by weight |
| (8) | magnesium stearate | 3 parts by weight |
| (9) | talc | 10 parts by weight |

### (Formulation Example 4)

The following components were granulated according to a conventional method and packed in a gelatin hard capsule. A capsule containing 20 mg of coenzyme Q₁₀ and 20 mg of pyrroloquinoline quinone in one capsule was obtained.

| | | |
|---|---|---|
| (1) | coenzyme Q₁₀ (reduced coenzyme Q₁₀:oxidized coenzyme Q₁₀=85:15) | 20 parts by weight |
| (2) | pyrroloquinoline quinone | 20 parts by weight |
| (3) | crystalline cellulose | 40 parts by weight |
| (4) | cornstarch | 20 parts by weight |
| (5) | lactose | 62 parts by weight |
| (6) | magnesium stearate | 2 parts by weight |
| (7) | polyvinylpyrrolidone | 3 parts by weight |

### (Formulation Example 5)

Olive oil was heated to 60°C, and coenzyme Q₁₀ (reduced coenzyme Q₁₀:oxidized coenzyme Q₁₀=85:15) similarly melted at 60°C and citric acid were added. Vitamin E was added thereto by small portions to homogenize the mixture, and the mixture was prepared into a soft capsule by a conventional method. A soft capsule preparation containing 20 mg of coenzyme Q₁₀ and 20 mg of citric acid in one capsule was obtained.

| | | |
|---|---|---|
| (1) | coenzyme Q₁₀ | 20 parts by weight |
| (2) | citric acid | 20 parts by weight |
| (3) | vitamin E | 15 parts by weight |
| (4) | olive oil | 350 parts by weight |

### (Formulation Example 6)

Coenzyme Q₁₀ (reduced coenzyme Q₁₀:oxidized coenzyme Q₁₀=85:15) and citric acid were added to acetone, and the mixture was adsorbed to crystalline cellulose (fine powder) and dried. This was mixed with cornstarch and the mixture was prepared into a powder by a conventional method.

| | | |
|---|---|---|
| (1) | coenzyme Q₁₀ | 10 parts by weight |
| (2) | citric acid | 10 parts by weight |
| (3) | crystalline cellulose | 40 parts by weight |
| (4) | cornstarch | 55 parts by weight |

### (Formulation Example 7)

Coenzyme Q₁₀ (reduced coenzyme Q₁₀:oxidized coenzyme Q₁₀=85:15) and citric acid were added to acetone, and the mixture was adsorbed to crystalline cellulose (fine powder) and dried. This was mixed with cornstarch, lactose, carboxymethylcellulose and magnesium stearate, an aqueous solution of polyvinylpyrrolidone was added as a binder and the mixture was granulated by a conventional method. Talc was added thereto as a lubricant, mixed, and the mixture was compressed to give a tablet containing 20 mg of coenzyme Q₁₀ and 20 mg of citric acid per tablet.

| | | |
|---|---|---|
| (1) | coenzyme Q₁₀ | 20 parts by weight |
| (2) | citric acid | 20 parts by weight |
| (3) | cornstarch | 25 parts by weight |
| (4) | lactose | 15 parts by weight |
| (5) | carboxymethylcellulose calcium | 10 parts by weight |
| (6) | crystalline cellulose | 40 parts by weight |
| (7) | polyvinylpyrrolidone | 5 parts by weight |
| (8) | magnesium stearate | 3 parts by weight |
| (9) | talc | 10 parts by weight |

### (Formulation Example 8)

The following components were granulated according to a conventional method and packed in a gelatin hard capsule. A capsule containing 20 mg of coenzyme Q₁₀ and 20 mg of citric acid in one capsule was obtained.

| | | |
|---|---|---|
| (1) | coenzyme Q₁₀ (reduced coenzyme Q₁₀:oxidized coenzyme Q₁₀=85:15) | 20 parts by weight |
| (2) | citric acid | 20 parts by weight |
| (3) | crystalline cellulose | 40 parts by weight |
| (4) | cornstarch | 20 parts by weight |
| (5) | lactose | 62 parts by weight |
| (6) | magnesium stearate | 2 parts by weight |
| (7) | polyvinylpyrrolidone | 3 parts by weight |

## Claims

1. A mitochondrial activator comprising pyrroloquinoline quinone or a derivative thereof.

2. The mitochondrial activator of claim 1, which comprises
(a) pyrroloquinoline quinone or a derivative thereof and
(b) a reduced coenzyme Q represented by the following formula (1); 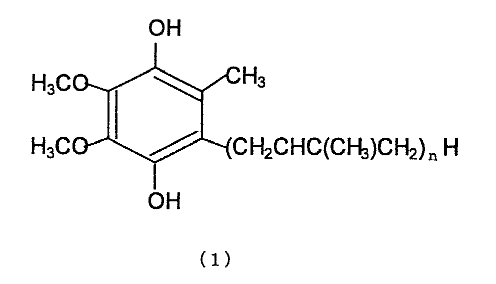 wherein n is an integer of 1 to 12, or
(c) an oxidized coenzyme Q represented by the following formula (2); 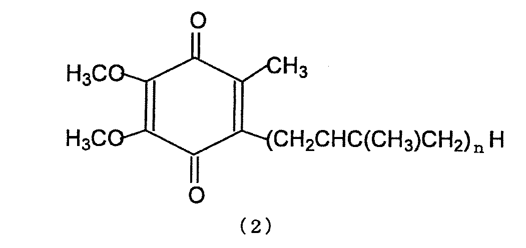
wherein n is an integer of 1 to 12.

3. The mitochondrial activator of claim 2, comprising at least
(a) pyrroloquinoline quinone or a derivative thereof, and
(b) a reduced coenzyme Q represented by the aforementioned formula (1).

4. The mitochondrial activator of claim 2, wherein the coenzyme Q is a coenzyme Q₁₀.

5. A composition comprising the mitochondrial activator of any one of claims 1 to 4 and an antioxidant.

6. The composition of claim 5, wherein the antioxidant is at least one kind of compound selected from the group consisting of vitamin C, a vitamin C derivative, probucol, lycopene, vitamin A, carotenoids, vitamin B, a vitamin B derivative, flavonoids, polyphenols, glutathione, selenium, sodium thiosulfate, vitamin E, a vitamin E derivative, pycnogenol, flavangenol, citric acid, a citric acid derivative, superoxide dismutase (SOD), glutathioneperoxidase, glutathione-S-transferase, glutathione reductase, catalase and ascorbate peroxidase.

7. A pharmaceutical or food composition comprising the mitochondrial activator of any one of claims 1 to 4 and a carrier acceptable as a pharmaceutical agent or food.

8. A composition for the prophylaxis or treatment of a disease caused by mitochondrial dysfunction, which comprises an effective amount of the mitochondrial activator of any one of claims 1 to 4.

9. The composition of claim 8, wherein the disease is selected from the group consisting of diabetes, a mitochondrial disease and a brain disease.

10. A method of preventing or treating a disease caused by mitochondrial dysfunction, which comprises administering an effective amount of the mitochondrial activator of any one of claims 1 to 4 to a mammal.

11. The method of claim 10, wherein the disease is selected from the group consisting of diabetes, a mitochondrial disease and a brain disease.

12. A production method of the mitochondrial activator of any one of claims 1 to 4, which comprises mixing
(a) pyrroloquinoline quinone or a derivative thereof, and
(b) a reduced coenzyme Q represented by the aforementioned formula (1) and/or
(c) an oxidized coenzyme Q represented by the aforementioned formula (2).

13. A production method of the composition for the prophylaxis or treatment of the disease caused by mitochondrial dysfunction of claim 8, which comprises mixing
(a) pyrroloquinoline quinone or a derivative thereof, and
(b) a reduced coenzyme Q represented by the aforementioned formula (1) and/or
(c) an oxidized coenzyme Q represented by the aforementioned formula (2).

14. A mitochondrial activator comprising
(b) a reduced coenzyme Q represented by the below-mentioned formula (1): 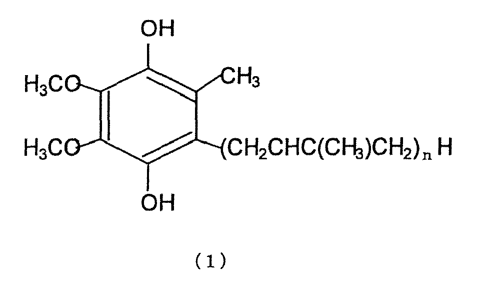 wherein n is an integer of 1 to 12, or
(c) an oxidized coenzyme Q represented by the following formula (2); 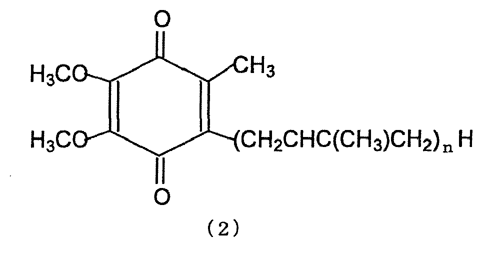 wherein n is an integer of 1 to 12, and
(d) citric acid or a derivative thereof.

15. The mitochondrial activator of claim 14, which comprises at least
(b) a reduced coenzyme Q represented by the aforementioned formula (1) and
(d) citric acid or a derivative thereof.

16. The mitochondrial activator of claim 14, wherein the coenzyme Q is a coenzyme Q₁₀.

17. A composition for a pharmaceutical agent or food, which comprises the mitochondrial activator of any one of claims 14 to 16, and a carrier acceptable as a pharmaceutical agent or food.

18. The composition of claim 17, further comprising an antioxidant substance.

19. The composition of claim 18, wherein the antioxidant is one or more kinds of compounds selected from the group consisting of vitamin C, a vitamin C derivative, probucol, lycopene, vitamin A, carotenoids, vitamin B, a vitamin B derivative, flavonoids, polyphenols, glutathione, selenium, sodium thiosulfate, vitamin E, a vitamin E derivative, pycnogenol, flavangenol, pyrroloquinoline quinone, a pyrroloquinoline quinone derivative, superoxide dismutase (SOD), glutathione peroxidase, glutathione-S-transferase, glutathione reductase, catalase, and ascorbate peroxidase.

20. A composition for the prophylaxis or treatment of a disease caused by mitochondrial dysfunction in a mammal, which comprises an effective amount of the mitochondrial activator of any one of claims 14 to 16.

21. The composition of claim 20, wherein the disease is selected from the group consisting of diabetes, a mitochondrial disease and a brain disease.

22. A method of preventing or treating a disease caused by mitochondrial dysfunction in a mammal, which comprises administering an effective amount of the mitochondrial activator of any one of claims 14 to 16 to the mammal.

23. The method of claim 22, wherein the disease is selected from the group consisting of diabetes, a mitochondrial disease and a brain disease.

24. A production method of the mitochondrial activator of any of claims 14 to 16, which comprises mixing
(b) a reduced coenzyme Q represented by the aforementioned formula (1) and/or
(c) an oxidized coenzyme Q represented by the aforementioned formula (2), and
(d) citric acid or a derivative thereof.

25. A production method of the composition for the prophylaxis or treatment of the disease caused by mitochondrial dysfunction of the aforementioned claim 20, which comprises mixing
(b) a reduced coenzyme Q represented by the aforementioned formula (1) and/or
(c) an oxidized coenzyme Q represented by the aforementioned formula (2), and
(d) citric acid or a derivative thereof.

26. A mitochondrial activator comprising citric acid or a derivative thereof.

27. A mitochondrial activator comprising
(a) pyrroloquinoline quinone or a derivative thereof and,
(b) a reduced coenzyme Q represented by the below-mentioned formula (1) 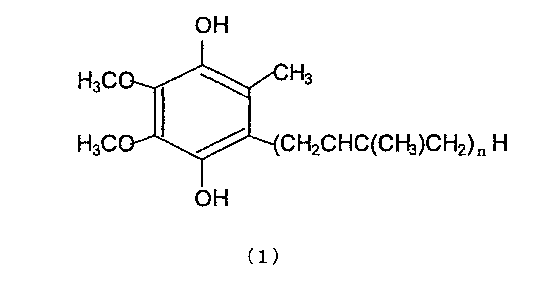 wherein n is an integer of 1 to 12, and/or
(c) an oxidized coenzyme Q represented by the following formula (2); 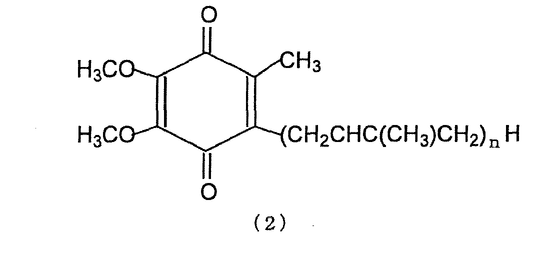 wherein n is an integer of 1 to 12, and
(d) citric acid or a derivative thereof.
